# EUROPEAN PATENT APPLICATION

(11) **EP 4 635 553 A2**
(43) Date of publication of application: **22.10.2025**
(21) Application number: 25193007.9
(22) Date of filing: 10.08.2023
(51) Int. Cl.: A61M 39/22

(54) **FLUID CONNECTOR DEVICE, BLOOD COLLECTION SYSTEM AND METHOD**

(30) Priority: 28.09.2022 US 202263411099 P
(62) Divisional of application: 23764478.6
(71) Applicant: CareFusion 303, Inc., San Diego, CA 92130 (US)
(72) Inventor: Khan, Mohammed Mehtab, 560045 Karnataka Bengaluru (IN); Jadhav, Amarsinh Deeliprao, 560045 Karnataka Bengaluru (IN); Austin, Abin, 560045 Karnataka Bengaluru (IN)
(74) Representative: Zenz Patentanwälte Partnerschaft mbB

(57) **Abstract**

A fluid connector device for a blood collection system is disclosed. In order to be safely used with different gauges, the fluid connector device can regulate blood flow therethrough based upon the catheter gauge. For example, when 18-to-22-gauge catheters are used, the fluid connector device regulates the blood flow by positioning a slider assembly (within the fluid connector device) to a setting that allows a single channel to transmit blood through the fluid connector device into a fluid inlet and out of a fluid outlet of the fluid connector device. Alternatively, in another example, when 24-gauge (or greater) catheters are used, the slider assembly can be actuated to a new position, which allows multiple channels to transmit blood into the fluid inlet and out of the fluid outlet. By regulating blood flow, the fluid connector device can limit or prevent hemolysis, while being universally used with different catheters.

## Description

### CROSS REFERENCE TO RELATED APPLICATION

This application claims the benefit of priority under 35 U.S.C. § 119 to Provisional Application for Patent Serial No. 63/411,099, entitled "PIVC-INTEGRATED HEMOLYSIS-REDUCTION ACCESSORIES FOR DIRECT BLOOD DRAW ON MULTIPLE NEEDLE GAUGES," filed on September 28, 2022, the entire contents of which is incorporated herein by reference.

### TECHNICAL FIELD

The present disclosure generally relates to blood draw and administration of parenteral fluids to a patient, and particularly to systems and methods to reduce hemolysis in PIVC blood draw.

### BACKGROUND

Catheters are commonly used for a variety of infusion therapies. For example, catheters may be used for infusing fluids, such as normal saline solution, various medicaments, and total parenteral nutrition, into a patient. Catheters may also be used for withdrawing blood from the patient.

A common type of catheter is an over-the-needle peripheral intravenous ("IV") catheter ("PIVC"). As its name implies, the over-the-needle catheter may be mounted over an introducer needle having a sharp distal tip. A catheter assembly may include a catheter hub, the catheter extending distally from the catheter hub, and the introducer needle extending through the catheter. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

For blood withdrawal or collecting a blood sample from a patient, a blood collection container may be used. The blood collection container may include a syringe. Alternatively, the blood collection container may include a test tube with a rubber stopper at one end. In some instances, the test tube has had all or a portion of air removed from the test tube so pressure within the test tube is lower than ambient pressure. Such a blood collection container is often referred to as an internal vacuum or a vacuum tube. The blood collection container may also be a VACUTAINER^{®} blood collection tube, available from Becton Dickinson & Company.

The blood collection container may be coupled to the catheter. When the blood collection container is coupled to the catheter, a pressure in the vein is higher than a pressure in the blood collection container, which pushes blood into the blood collection container, thus filling the blood collection container with blood. A vacuum within the blood collection container decreases as the blood collection container fills, until the pressure in the blood collection container equalizes with the pressure in the vein, and the flow of blood stops. Unfortunately, as blood is drawn into the blood collection container, red blood cells are in a high shear stress state and susceptible to hemolysis due to a high initial pressure differential between the vein and the blood collection container. Hemolysis may result in rejection and discard of a blood sample. The high initial pressure differential can also result in catheter tip collapse, vein collapse, or other complications that prevent or restrict blood from filling the blood collection container.

The description provided in the background section should not be assumed to be prior art merely because it is mentioned in or associated with the background section. The background section may include information that describes one or more aspects of the subject technology.

### SUMMARY

The present disclosure provides a fluid connector device, comprising a first connector, a second connector that combines with the first connector to form an internal chamber, the second connector comprising a post, a first opening formed in the post, and a second opening in the post, and a slider assembly disposed in the internal chamber, wherein the slider assembly i) in a first position, aligns with the first opening and seals the second opening, and ii) in a second position, aligns with the second opening and seals the first opening.

In some instances, the present disclosure provides a blood collection system, comprising a blood collection device, and a fluid connector device fluidly coupled to the blood collection device, the fluid connector device comprising a first connector, a second connector that combines with the first connector to form an internal chamber, the second connector the second connector comprising a post, a first opening formed in the post, and a second opening in the post, and a slider assembly disposed in the internal chamber, the slider assembly configured to deliver a fluid through the first opening or the second opening, based on a position of the slider assembly relative to the post.

The present disclosure also provides a method for regulating a fluid through a blood collection system, the method comprising, at a fluid connector device, receiving, by a slider assembly, the fluid from a first connector, receiving, at a first opening of a second connector, the fluid from the slider assembly, when the slider assembly is moved relative to the second connector, receiving, at a second opening of a second connector, the fluid from the slider assembly; and sealing the first opening from the fluid.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

The following figures are included to illustrate certain aspects of the embodiments, and should not be viewed as exclusive embodiments. The subject matter disclosed is capable of considerable modifications, alterations, combinations, and equivalents in form and function, as will occur to those skilled in the art and having the benefit of this disclosure.
FIG. 1 illustrates a collection system, in accordance with some aspects of the present disclosure.
FIG. 2 illustrates an exploded view of the fluid connector device shown in FIG. 1, in accordance with some aspects of the present disclosure.
FIG. 3 illustrates a perspective view of the component of the slider assembly shown in FIG. 2, showing additional features of the component, in accordance with aspects of the present disclosure.
FIG. 4 illustrates a perspective view of the fluid connector device, showing features of the slider assembly in an initial position, in accordance with some aspects of the present disclosure.
FIG. 5 illustrates a partial cross-sectional view of the fluid connector device, showing an opening in the slider assembly aligned with a respective opening in the connector, in accordance with some aspects of the present disclosure.
FIG. 6 illustrates an alternative partial cross-sectional view of the fluid connector device, showing the respective openings in the slider assembly and the connector aligned with each other, in accordance with some aspects of the present disclosure.
FIG. 7 illustrates a perspective view of the fluid connector device, showing the slider assembly move to an alternate position, in accordance with some aspects of the present disclosure.
FIG. 8 illustrates a partial cross-sectional view of the fluid connector device, showing openings in the slider assembly aligned with respective openings in the connector, in accordance with some aspects of the present disclosure.
FIG. 9 illustrates an alternative partial cross-sectional view of the fluid connector device, showing the openings in the slider assembly aligned with the respective openings in the connector, in accordance with some aspects of the present disclosure.
FIG. 10 illustrates a flowchart showing a method for regulating a fluid through a blood collection system, in accordance with some aspects of the present disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions may be provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Blood draw via a vascular access device has drawn increasing attention attributed to minimized needle sticks and improved operation efficiency as compared with traditional blood draw methods with venipuncture. Current blood draw using a peripheral intravenous catheter (PIVC) has seen some challenges, one of the most critical is hemolysis related blood quality. In particular, with currently existing PIVC products in the market, along with the standard connection (such as a short extension set and a needleless connector), and blood collection devices (such as a Vacutainer), the shear stress exerted onto blood cells tends to be on the verge of hemolyzing.

Various embodiments of the present disclosure are directed to providing systems and methods to address hemolysis in PIVC blood draw with a hemolysis reduction accessory (also referred to herein as a fluid connector device) which is pre-attached to the PIVC and serves as a flow regulator or flow restrictor to reduce risk of hemolysis. The hemolysis-reduction accessory is advantageously compatible with PIVC placement and does not necessitate change to any of the existing operations. The hemolysis-reduction accessory of the various embodiments described herein is potentially applicable to a wide variety of PIVC products, and compatible with existing blood collection devices and infusion disposables.

Various embodiments of the present disclosure focus on effective flow restriction with the add-on hemolysis-reduction accessory (also referred to herein as a fluid connector device) that regulates the overall flow rate of the entire fluid path as blood cells travel through. The fluid connector device can be either assembled with the PIVC or co-packaged with the PIVC. As such, there is no additional operation during catheter placement since the device has a vented lumen that enables blood flashback. The clinician may connect a blood collection device to the port of the accessory and can then draw blood to the intended volume. After blood draw, the clinician may disconnect and discard the fluid connector device and the blood collection device together. As such, this fluid connector device can be either for single blood draw or stay inline throughout indwell.

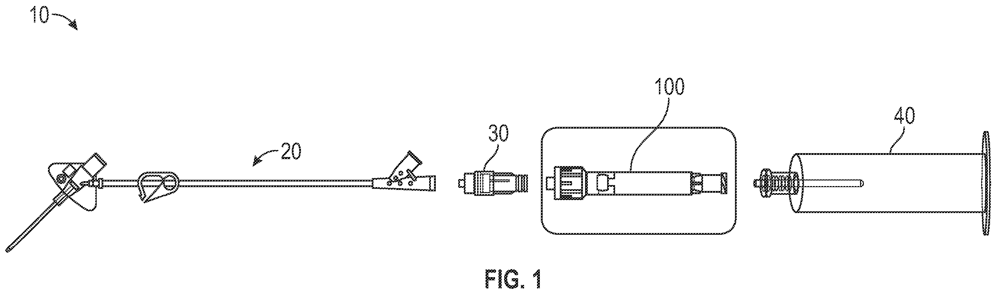

FIG. 1 illustrates a collection system 10, in accordance with some aspects of the present disclosure. The collection system 10 is designed to collect fluid (e.g., blood) from a patient. Accordingly, the collection system 10 may be referred to as a blood collection system. The collection system 10 can take the form of a vascular access device with a PIVC assembly. As shown, the collection system 10 include a catheter assembly 20. In some embodiments, the catheter assembly 20 may include or correspond to any suitable catheter assembly. In some embodiments, the catheter assembly 20 may be integrated with and include an extension tube, which may extend from and be integrated with a side port of the catheter hub. A non-limiting example of an integrated catheter assembly is the BD NEXIVA^{™} Closed IV Catheter system, available from Becton Dickinson and Company. In some embodiments, a proximal end of the extension tube may be coupled to an adapter, such as, for example, a Y-adapter or single port luer adapter.

Also, the collection system 10 may include a connector 30 that connects to the catheter assembly 20. In order to collect fluid (e.g., blood) from a patient, the collection system 10 further includes a collector 40. When the collected fluid is blood from a patient, the collector 40 may be referred to as a blood collector.

Additionally, the collection system 10 includes a fluid connector device 100, in accordance with some aspects of the present disclosure. The fluid connector device 100 may be configured to reduce a likelihood of hemolysis during blood collection using the collection system 10. In some embodiments, the fluid connector device 100 may include a distal end, which may include a body or distal connector configured to couple to the catheter assembly 20 by way of the connector 30. The connector 30 may include a male luer connector, or another suitable connector.

In some embodiments, the distal connector of fluid connector device 100 may be configured to couple to the Y-adapter of the catheter assembly 20 without the use of the connector 30. In some embodiments, the catheter assembly 20 may be non-integrated and may not include the extension tube. In these and other embodiments, the fluid connector device 100 may be configured to couple to the proximal end of the catheter hub or another suitable portion of the catheter assembly. In some embodiments, the catheter assembly 20 may be coupled to a removable extension tube. In some embodiments, the fluid connector device 100 may be coupled directly to the catheter adapter, eliminating the extension tube and providing a compact catheter system.

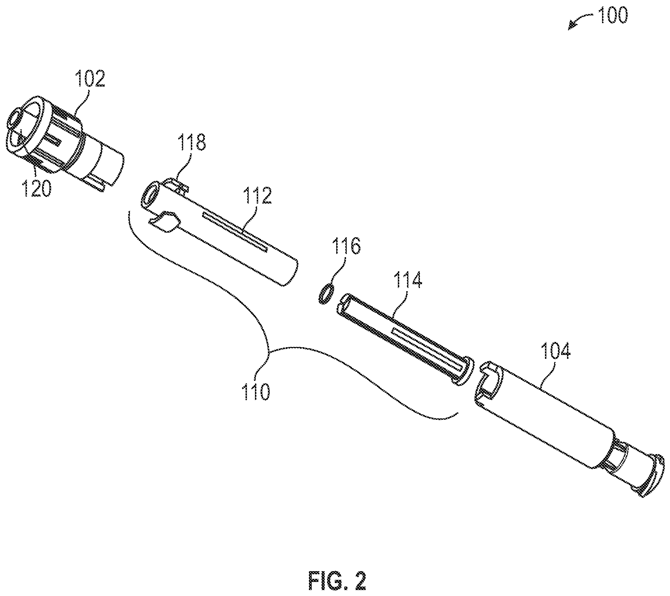

FIG. 2 illustrates an exploded view of the fluid connector device 100 shown in FIG. 1, in accordance with some aspects of the present disclosure. The fluid connector device 100 may include a connector 102 configured to couple to the connector 30 of the collection system 10 (shown in FIG. 1). Additionally, the fluid connector device 100 may include a connector 104 configured to couple with the collector 40 of the collection system 10. The connector 102 and the connector 104 may be referred to as a first connector and a second connector, respectively. However, "first" and "second" may be used interchangeably. The connectors 102 and 104 may combine to form an internal chamber, or internal volume or space.

Also, the fluid connector device 100 includes slider assembly 110 disposed partially within the connector 102 and partially within the connector 104 (i.e., the internal chamber). As shown, the slider assembly 110 includes a component 112 and a component 114. The component 112 and the component 114 may be referred to as a first component and a second component, respectively. However, "first" and "second" may be used interchangeably. In order to provide a seal between the components 112 and 114 and prevent leakage therebetween, the slider assembly 110 includes a ring 116. In some embodiments, the ring 116 is an O-ring. The slider assembly 110 is designed to act as a fluid regulator or flow restrictor through the fluid connector device 100. For example, the fluid connector device 100 can be integrated with various vascular access devices, each of which may include a different gauge (G). Using the slider assembly 110, the fluid connector assembly 100 can provide the safe collection of blood by limiting the blood flow in applications using catheter assemblies approximately in the range of 18G to 22G, while allowing for increased (or relatively higher) blood flow in applications with catheter assemblies with 24G or higher. To regulate fluid flow, the slider assembly 110 is designed to move relative to the connectors 102 and 104. In this regard, the slider assembly 110 includes an interface 118 designed for user interaction to actuate the slider assembly 110. The interface 118, integrated with the component 112, extends radially outward relative to the connectors 102 and 104.

The connector 102 may take the form of a male luer that includes a proximal end for connecting to the slider assembly 110, and a distal end including a second connection portion for connecting to a needleless connector. The first connection portion may have an inner surface defining a lumen 120 therethrough for coupling to the slider assembly 110. Also, the connector 104 may take the form of a female luer that includes a proximal end for connecting to the slider assembly 110, and a distal end including a second connection portion for connecting to a collector. The connector 104 may have an inner surface defining a lumen (not shown) therethrough for coupling to the slider assembly 110. Accordingly, the connectors 102 and 104 can fluidly connect the fluid connector device 100 to fluid drawing components.

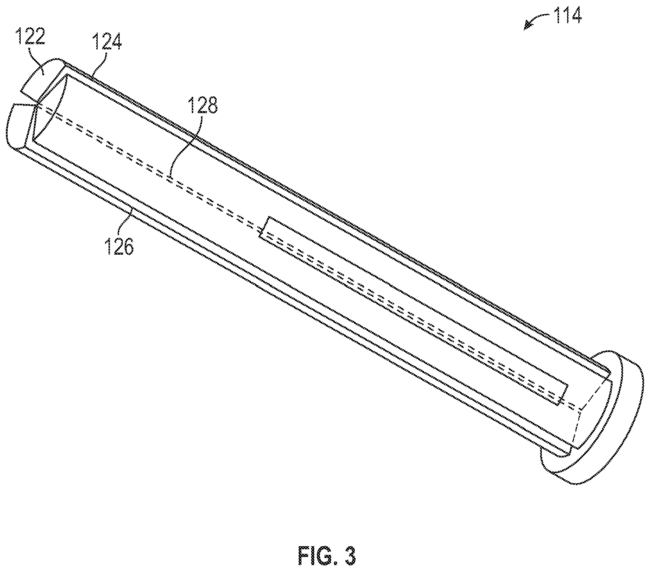

FIG. 3 illustrates a perspective view of the component 114 of the slider assembly 110 shown in FIG. 2, showing additional features of the component 114, in accordance with aspects of the present disclosure. The component 114 may include a cylindrical body 122, or at least substantially cylindrical body. Also, the component 114 may include multiple indentations running lengthwise along a major axis of the cylindrical body 122. For example, the component 114 includes an indentation 124, an indentation 126, and an indentation 128 (shown as dotted lines). The indentations 124, 126, and 128 form, in part, individual fluid paths along the cylindrical body 122. As will be shown in further detail below, each of the indentations 124, 126, and 128 combine with a respective indentation in the component 112 to form channels, or blood draw channels.

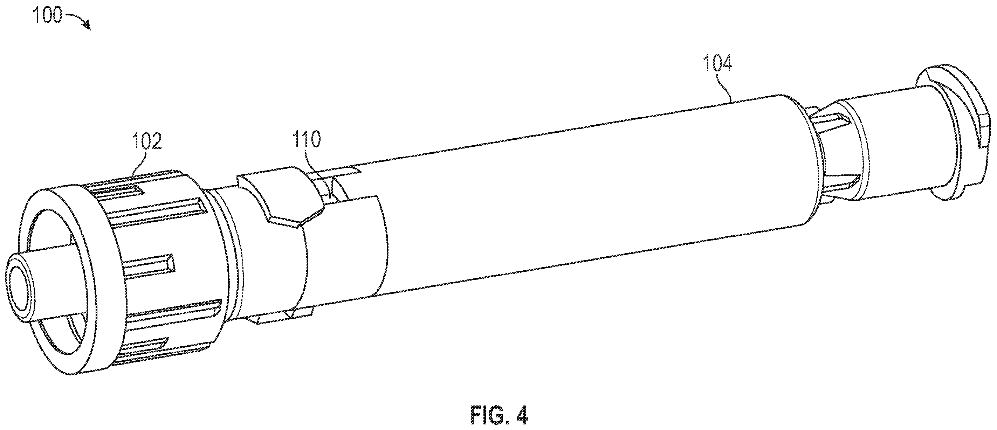

FIG. 4 illustrates a perspective view of the fluid connector device 100, in accordance with some aspects of the present disclosure. As shown, the fluid connector device 100 in an initial position, or first position, based on the position of the slider assembly 110 relative to the connectors 102 and 104. The position of the slider assembly 110 is suitable for use with catheters having gauges that are in the size range of 18G-22G, which cover several catheter applications.

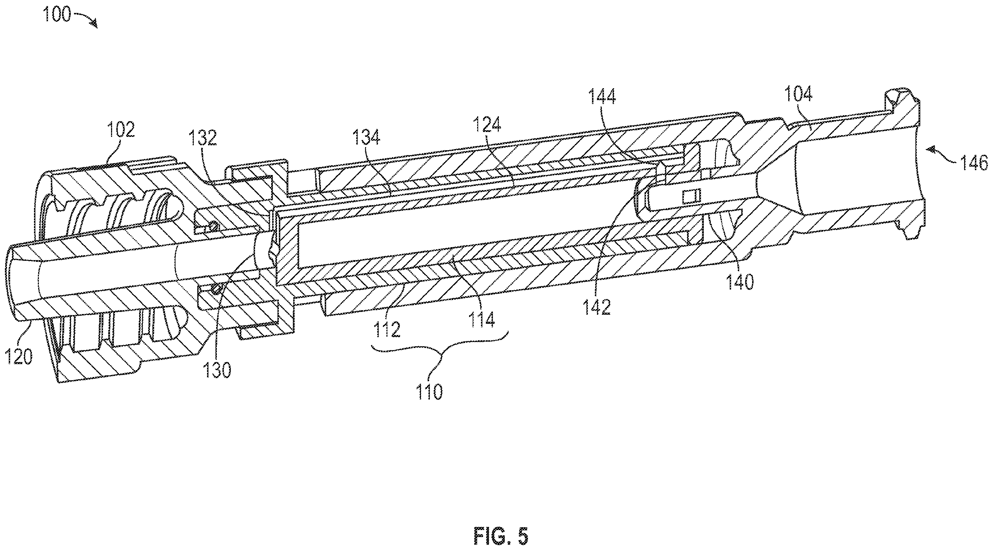

FIG. 5 illustrates a partial cross-sectional view of the fluid connector device 100, showing an opening in the slider assembly 110 aligned with a respective opening in the connector 104, in accordance with some aspects of the present disclosure. The lumen 120 of the connector 102 provides a fluid inlet for the fluid connector device 100. As fluid enters the connector 102, the fluid can pass through an opening 130 of the component 112, where the fluid can subsequently flow into a channel 132 formed by the indentation 124 of the component 114 and an indentation 134 of the component 112. Accordingly, the channel 132 is fluidly coupled to the lumen 140 in the initial/first position of the slider assembly 110. As shown, the indentations 124 and 134 are aligned to form the channel 132. Additional channels (not shown in FIG. 5) are also present, and fluid may further flow into these additional channels.

While fluid may flow through multiple channels formed by the components 112 and 114, based on the position of the slider assembly 110 relative to the connectors 102 and 104, fluid within a channel (i.e., the channel 132) may enter the connector 104, while the remaining channels are sealed off from allowing fluid to pass into the connector 104. In this regard, the connector 104 includes a lumen 140 that includes an opening 142. Additionally, the component 114 includes an opening 144. Each of the openings 142 and 144 forms a radial opening in the lumen 140 and the component 114, respectively. Also, as shown in FIG. 5, the openings 142 and 144 are aligned with each other, thereby allowing fluid in the channel 132 to pass through the openings 142 and 144, and exit the connector 104 via a fluid outlet 146 of the connector 104. The fluid outlet 146 may form a fluid outlet for the fluid connector device 100.

In some embodiments of the present disclosure, the connector 104 includes a post 105 that extends into the lumen 106 formed by the inner surface of the connector 104. The post 105 forms a portion of a fluid pathway through the connector 104. At least a portion of the fluid pathway through the post 105 can be formed by the opening 142 through the post. In some aspects, the post 105 includes more than one opening extending therethrough, and in some embodiments, the post 105 includes three, four, or more openings 142, 143, 145. Any of the openings through the post 105 can extend radially outward, relative to a longitudinal axis of the connector 104, to form the portion of the fluid pathway between the lumen 106 and the fluid outlet 146.

Still referring to FIG. 5, the post 105 includes a first opening 142 and a second opening 143, where the first and second openings 142 and 143 are aligned along a longitudinal axis that is approximately parallel to a longitudinal axis formed by the post. In some embodiments of the present disclosure, the third opening 145 radially offset relative to the first and second openings 142 and 143 by an angle around the longitudinal axis formed by the post 105.

When the slider assembly 110 is coupled with the connector 104, the post 105 can extend into any of the components 112 and 114 forming the slider assembly. The slider assembly 110, in some aspects, is longitudinally movable or slidable relative to the post 105. The post 105 can be partially positioned or extend into the slider assembly 110, which can permit any of the components 112 and 114 of the slider assembly to move along the post 105, relative to the connector 104. Although, in some embodiments, it is contemplated that the slider assembly 110 is rotatable around the post 105, it is also contemplated in the present disclosure, that the slider assembly 110 is rotationally coupled or fixed with the post 105.

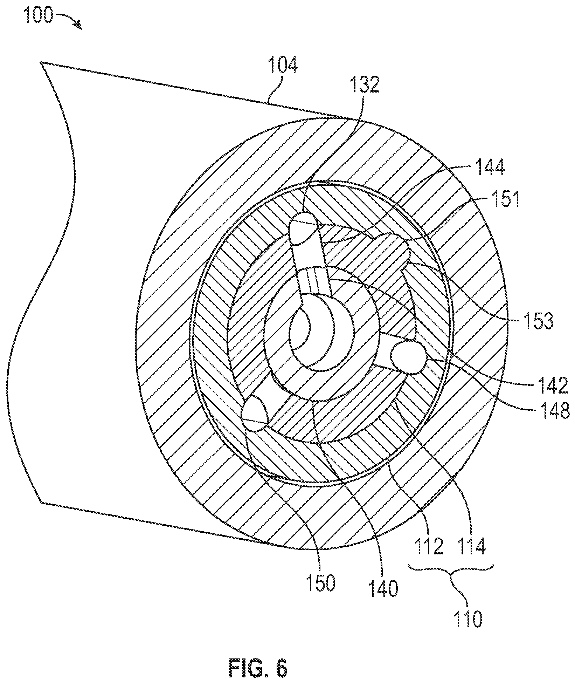

FIG. 6 illustrates an alternative partial cross-sectional view of the fluid connector device 100, showing the respective openings in the slider assembly 110 (i.e., component 114 of the slider assembly 110) and the connector 104 aligned with each other, in accordance with some aspects of the present disclosure. As shown, the channel 132 opens to the opening 144 of the component 114. Based on the alignment between the openings 142 and 144, fluid flowing through the channel 132 enters the lumen 140 of the connector 104, and flows out of the connector 104 via the fluid outlet 146 (shown in FIG. 5).

As further illustrated in FIG. 6, there are additional channels formed in the components 112 and 114. For example, a channel 148 and a channel 150 (both similar to the channel 132) are formed by respective indentations in the components 112 and 114. The channel 132, the channel 148, and the channel 150 may be referred to as a first channel, a second channel, and a third channel, respectively. However, "first," "second," and "third" may be used interchangeably. Also, each of the channels 132, 148, and 150 may be referred to as a blood draw channel. While fluid can flow through the channels 148 and 150, fluid will now flow into the lumen 140 of the connector 104 based upon the position of the slider assembly 110, as a respective opening in the lumen 140 is not aligned with openings in the component 114. Accordingly, in the initial/first position of the slider assembly 110, only the channel 132 can deliver fluid into the connector 104 that exits the fluid connector device 100 via the fluid outlet 146.

Additionally, in order to couple the components 112 and 114 of the slider assembly 110 together to properly align the indentations to form the channels, the components 112 and 114 may include alignment features. For example, as shown in FIG. 6, the component 112 includes an indentation 151 and the component 114 includes a protrusion 153 that fits into the indentation 151. The indentation 151 and the protrusion 153 may each run lengthwise along the component 112 and the component 114, respectively. When the component 114 is inserted into the component 112, the component 114 can be rotated, relative to the component 112, to align the protrusion 153 with the indentation 151 and properly position the component 114 in the component 112 to form the desired channels.

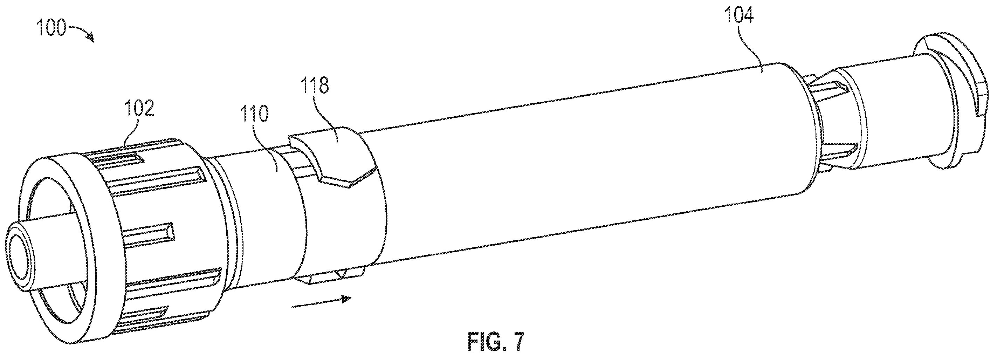

FIG. 7 illustrates a perspective view of the fluid connector device 100, showing the slider assembly 110 move to an alternate position, in accordance with some aspects of the present disclosure. As shown, the fluid connector device 100 in a subsequent position, or second position, based on the position of the slider assembly 110 relative to the connectors 102 and 104. In this regard, by engaging the interface 118, the slider assembly 110 can be actuated relative to the connectors 102 and 104 in the direction of the arrow. The position of the slider assembly 110 is suitable for use with catheters having gauges that 24G (covering several pediatric catheter applications), and in some cases greater than 24G.

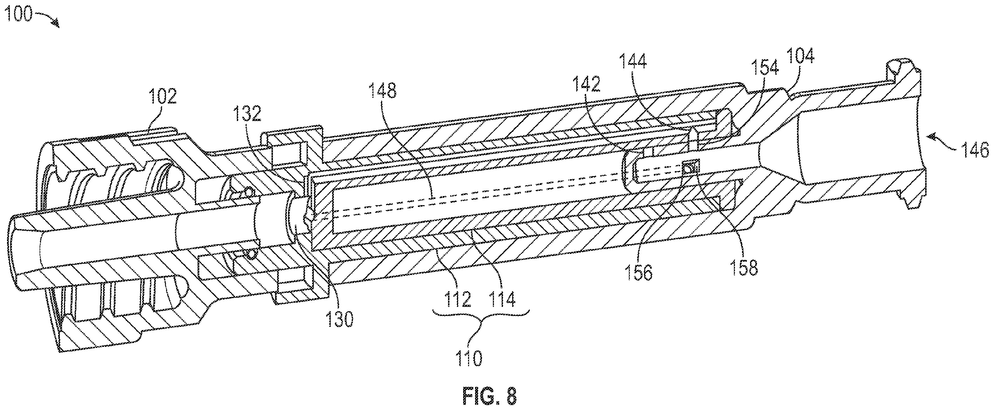

FIG. 8 illustrates a partial cross-sectional view of the fluid connector device 100, showing an opening in the slider assembly 110 aligned with a respective opening in the connector 104, in accordance with some aspects of the present disclosure. Fluid flowing through the channel 132 from the opening 130 of the component 112 can flow into the connector 104 via additional openings in the component 114 and the lumen 140 of the connector 104. As shown, the lumen 140 includes an opening 154, representing an additional, radial opening formed in the lumen 140. Based upon the movement of the slider assembly 110 relative to the connectors 102 and 104 (in particular, the connector 104), the openings 144 and 154 are aligned with each other, thereby allowing fluid in the channel 132 to pass through the openings 144 and 154 and into the lumen 140, where the fluid exits the connector 104 via the fluid outlet 146 of the connector 104. Moreover, due to the relative movement of the slider assembly 110, the openings 142 and 144 are no aligned with each other, thus preventing fluid in the channel 132 from entering the lumen 140 via the openings 142 and 144. When the openings 142 and 144 are not aligned, the engagement between the lumen 140 and the component 114 provides a fluid seal, thus preventing fluid from entering the opening 142.

Additionally, fluid entering the channel 148 can also enter the lumen 140. In this manner, the lumen 140 includes an opening 156 and the component 114 includes an opening 158. The openings 156 and 158 each represent an additional, radial opening formed in the lumen 140 and the component 114, respectively. As shown in FIG. 8, the openings 156 and 158 are aligned with each other, thereby allowing fluid in the channel 148 to pass through the openings 156 and 158 and into the lumen 140, where the fluid exits the connector 104 via the fluid outlet 146 of the connector 104.

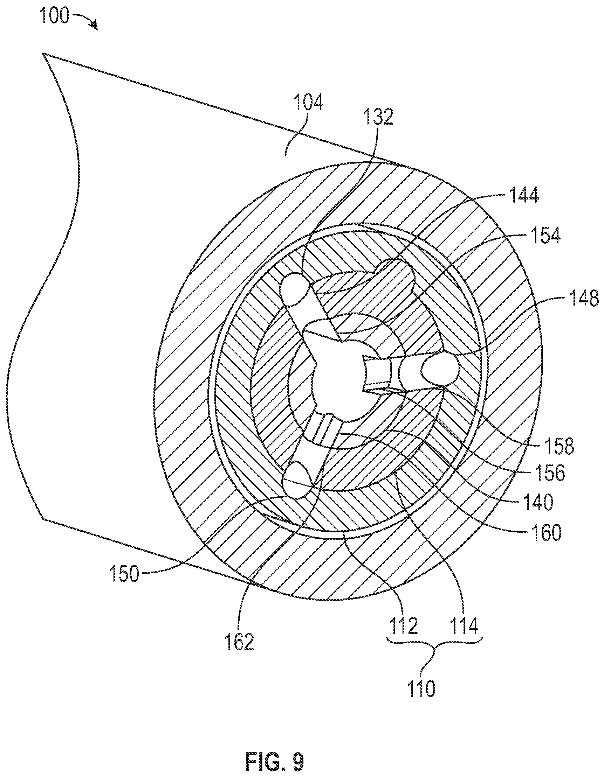

FIG. 9 illustrates an alternative partial cross-sectional view of the fluid connector device 100, showing the respective openings in the slider assembly 110 (i.e., component 114 of the slider assembly 110) and the connector 104 aligned with each other, in accordance with some aspects of the present disclosure. As shown, the channel 132 opens to the opening 144 of the component 114. Based on the alignment between the openings 144 and 154, fluid flowing through the channel 132 enters the lumen 140 of the connector 104, and flows out of the connector 104 via the fluid outlet 146 (shown in FIG. 8). Also, the channel 148 opens to the opening 158 of the component 114. Based on the alignment between the openings 156 and 158, fluid flowing through the channel 148 enters the lumen 140 of the connector 104, and flows out of the connector 104 via the fluid outlet 146 (shown in FIG. 8). Additionally, the lumen 140 includes an opening 160 and the component 114 includes an opening 162. The openings 160 and 162 each represent an additional, radial opening formed in the lumen 140 and the component 114, respectively. The channel 150 opens to the opening 162 of the component 114. Based on the alignment between the openings 160 and 162, fluid flowing through the channel 150 enters the lumen 140 of the connector 104, and flows out of the connector 104 via the fluid outlet 146 (shown in FIG. 8). Further, when the slider assembly 110 is moved back to the initial/first position (shown in FIG. 4-6), the engagement between the lumen 140 and the component 114 provides a fluid seal, thus preventing fluid from entering the openings 154, 156, and 158.

Accordingly, the slider assembly 110, in the second positions, provides additional fluid paths (three) as compared to the single fluid path of the slider assembly 110 in the first position. The slider assembly 110 provides the fluid connector device 100 with flow restricting capabilities, thus allowing the fluid connector device 100 to work with medical device applications, which require different sizes/gauges.

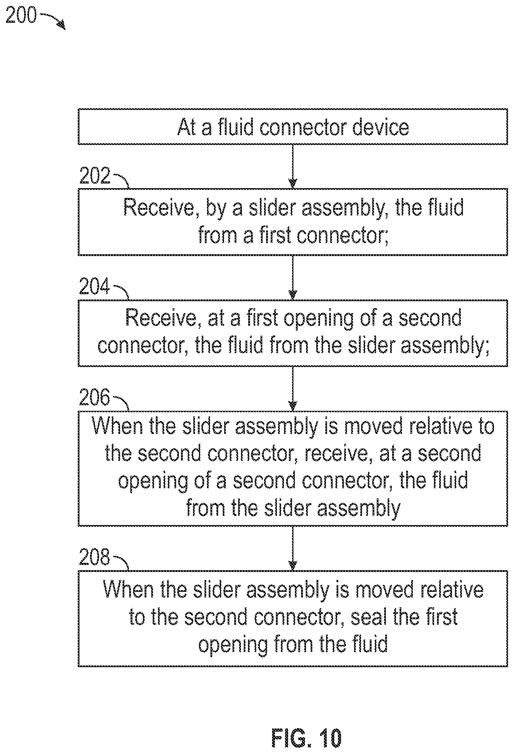

FIG. 10 illustrates a flowchart 200 showing a method for regulating a fluid through a blood collection system. The steps of the flowchart 200 may be performed by fluid connector devices described herein. While performing one or more steps of the flowchart 200, a fluid connector device may be part of a blood collection system that also includes, for example, a catheter and a collector for collection of fluid.

In step 202, a slider assembly receives the fluid from a first connector. The slider assembly may include one or more components, each of which are disposed (or at least substantially disposed) in the fluid connector device. Further, the slider assembly can be actuated to multiple position to regulate fluid flow through the fluid connector device.

In step 204, a first opening of a second connector receives the fluid from the slider assembly. The first opening may be formed in a lumen of the second connector. Based on a position of the slider assembly, the first opening of the second connector is aligned with an opening of the slider assembly (or a component thereof). Further, the slider assembly may include multiple channels, one of which is fluidly connected to the opening of the slider assembly and the first opening of the second connector. Moreover, the one fluidly connected opening may represent the only channel that can provide the fluid to the connector, and several other openings may be sealed from receiving the fluid. This configuration is based upon a position of the slider assembly.

In step 206, when the slider assembly is moved relative to the second connector, a second opening of a second connector receives the fluid from the slider assembly. In addition to the first opening, the second may be formed in the lumen of the second connector. Several additional openings similar to the second opening may also be formed in the lumen. Also, the slider assembly may include multiple channels, each of which is aligned with and fluidly coupled to at least one opening in the lumen. Moreover, based upon the position of the slider assembly, when moved, the second opening, as well as additional similar openings, may receive the fluid.

In step 208, when the slider assembly is moved relative to the second connector, the first opening is sealed from the fluid. While the first opening is sealed, the second opening, as well as the aforementioned additional openings, may receive the fluid. Accordingly, the flowchart 200 provide a method for limiting the number of channels that provide fluid through the fluid connector device, while also increasing the number of channels based on movement of the slider assembly.

The fluid connector devices and associated blood collection systems of the various embodiments described herein additionally provide further advantages over currently existing blood collection systems. For example, add-on fluid connector devices described herein allow for hemolysis-reduction function to be integrated for PIVC blood draw. Further, the fluid connector devices described herein are compatible with PIVC placement and allow for seamless blood draw at insertion. Ion some embodiments, the fluid connector devices have the potential to stay inline throughout PIVC indwell for multiple blood draws. Additionally, since the fluid connector devices are an add-on which can be easily incorporated without any changes to existing PIVC, there is minimal impact to clinical setting and operations.

The fluid connector device of the various embodiments described herein is thus advantageous over currently existing PIVC blood draw accessories/connectors because it is capable of operating effectively on multiple catheter gauge sizes. Accordingly, the fluid connector device of the various embodiments described herein may be referred to as a universal fluid connector device since the design is capable of being used across a wide range of catheter sizes (18G to 24+G) and catheter brands. The fluid connector device of the various embodiments described herein thus improves over currently existing PIVC blood draw accessories obviating the need to have different blood draw accessories/connectors for different catheter gauge range by providing a single universal connector which may show effective results across the varying catheter gauge ranges and brands. The fluid connector device of the various embodiments described herein aims to deliver the same performance on all the catheter gauges. By incorporating switchable blood flow paths in the form of the sliding assembly having multiple blood draw paths, fluid connector device of the various embodiments described herein can effectively work with 18G-22G gauge and other pediatric gauges. In contrast, with currently existing PIVC blood draw connectors/accessories, each design or concept is created to be compatible with a specific range of catheter gauge sizes in order to achieve satisfactory blood draw performance.

In accordance with various embodiments of the present disclosure, the channels (e.g., channels 132, 148, and 150) may each be in the form of a micro-channel fluid pathway through which fluid (e.g., blood) flows from the distal connector to the proximal connector for collection in the fluid collection device. For example, each blood draw channel in the form of a micro-channel along which fluid (e.g., blood) may flow from the distal connector into the proximal connector for collection into the fluid collection device may have a diameter in the range of approximately 0.01 inches to approximately 0.1 inches. A length of the micro-channel may can be in the range of approximately 0.5 inches to approximately 5 inches. In some instances, the micro-channel comprises a diameter of 0.025 inches and length of 1 inch. In some instances the diameter is 0.025 inches and the length is 0.75 inches. In some instances the diameter is 0.025 inches and the length is 1.5 inches. In some instances the diameter is 0.03 inches and the length is 3 inches. Accordingly, during blood collection or draw from the patient, the blood may flow into the blood collection device via either one or all three of the micro-channels of the first, second, and third blood draw channels having minimal diameter.

The subject technology is illustrated, for example, according to various aspects described below. Various examples of aspects of the subject technology are described as numbered clauses (1, 2, 3, etc.) for convenience. These are provided as examples and do not limit the subject technology. It is noted that any of the dependent clauses may be combined in any combination, and placed into a respective independent clause, e.g., clause 1 or clause 5. The other clauses can be presented in a similar manner.

Clause 1. A fluid connector device, comprising: a first connector; a second connector that combines with the first connector to form an internal chamber, the second connector comprising: a post, a first opening formed in the post, and a second opening in the post; and a slider assembly disposed in the internal chamber, wherein the slider assembly i) in a first position, aligns with the first opening and seals the second opening, and ii) in a second position, aligns with the second opening and seals the first opening.

Clause 2. The fluid connector device according to Clause 1, wherein the slider assembly comprises a channel fluidly connected to the first opening and the second opening.

Clause 3. The fluid connector device according to any one of Clauses 1 or 2, further comprising: a third opening formed in the post; and a fourth opening formed in the post, wherein the slider assembly, in the second position, aligns with the third opening and the fourth opening.

Clause 4. The fluid connector device according to Clause 3, wherein the slider assembly further comprises: a first channel fluidly connected to i) the first opening when the slider assembly is in the first position and ii) the second opening in the second position; a second channel fluidly connected to the third opening in the second position; and a third channel fluidly connected to the fourth opening in the second position.

Clause 5. The fluid connector device according to Clause 4, wherein the slider assembly further comprises: a first component; and a second component, wherein the first component and the second component form the first channel, the second channel, and the third channel.

Clause 6. The fluid connector device according to any one of Clauses 1 to 5, wherein the post is at least partially positioned in the slider assembly.

Clause 7. The fluid connector device according to any one of Clauses 1 to 5, the slider assembly is rotationally coupled with the post.

Clause 8. A blood collection system, comprising: a blood collection device; and a fluid connector device fluidly coupled to the blood collection device, the fluid connector device comprising: a first connector; a second connector that combines with the first connector to form an internal chamber, the second connector the second connector comprising: a post, a first opening formed in the post, and a second opening in the post; and a slider assembly disposed in the internal chamber, the slider assembly configured to deliver a fluid through the first opening or the second opening, based on a position of the slider assembly relative to the post.

Clause 9. The blood collection system according to Clause 8, wherein: when the slider assembly allows the fluid to pass through the first opening, the slider assembly seals the second opening, and when the slider assembly allows the fluid to pass through the second opening, the slider assembly seals the first opening.

Clause 10. The blood collection system according to any one of Clauses 8 or 9, wherein the slider assembly comprises: a first component; and a second component, wherein the first component and the second component form a channel fluidly connected to at least one of the first opening and the second opening.

Clause 11. The blood collection system according to any one of Clauses 8 to 10, wherein the slider assembly is moveable with respect to the first connector and the second connector.

Clause 12. The blood collection system according to any one of Clauses 8 to 11, further comprising: a third opening formed in the post; a fourth opening formed in the post, wherein the slider assembly is configured to deliver the fluid through the first opening or the third opening and the fourth opening, based on the position of the slider assembly.

Clause 13. The blood collection system according to any one of Clauses 8 to 12, wherein the slider assembly is movable relative to the first connector and the second connector.

Clause 14. The blood collection system according to Clause 13, wherein: the slider assembly comprises an interface, and the slider assembly is movable based on actuation of the interface.

Clause 15. A method for regulating a fluid through a blood collection system, the method comprising, at a fluid connector device: receiving, by a slider assembly, the fluid from a first connector; receiving, at a first opening of a second connector, the fluid from the slider assembly; when the slider assembly is moved relative to the second connector: receiving, at a second opening of a second connector, the fluid from the slider assembly; and sealing the first opening from the fluid.

Clause 16. The method of Clause 15, further comprising prior to the slider assembly being moved, sealing the second opening from the fluid.

Clause 17. The method to any one of Clauses 15 or 16, further comprising when the slider assembly is moved relative to the second connector, receiving, at a third opening and a fourth opening of the second connector, the fluid from the slider assembly.

Clause 18. The method of Clause 17, further comprising: providing, by a first channel, the fluid to the second opening; providing, by a second channel, the fluid to the second opening; and providing, by a third channel, the fluid to the fourth opening.

Clause 19. The method of Clause 18, further comprising receiving, only by the first channel, the fluid into the second connector when the second opening is sealed.

Clause 20. The method of Clause 18, further comprising receiving, by the first channel, the second channel, and the third channel, the fluid into the second connector when the first opening is sealed.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent. As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

It is understood that the specific order or hierarchy of steps, or operations in the processes or methods disclosed are illustrations of exemplary approaches. Based upon implementation preferences or scenarios, it is understood that the specific order or hierarchy of steps, operations or processes may be rearranged. Some of the steps, operations or processes may be performed simultaneously. In some implementation preferences or scenarios, certain operations may or may not be performed. Some or all of the steps, operations, or processes may be performed automatically, without the intervention of a user. The accompanying method claims present elements of the various steps, operations or processes in a sample order, and are not meant to be limited to the specific order or hierarchy presented.

All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112 (f) unless the element is expressly recited using the phrase "means for" or, in the case of a method claim, the element is recited using the phrase "step for." Furthermore, to the extent that the term "include," "have," or the like is used, such term is intended to be inclusive in a manner similar to the term "comprise" as "comprise" is interpreted when employed as a transitional word in a claim.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

The claims are not intended to be limited to the aspects described herein, but are to be accorded the full scope consistent with the language of the claims and to encompass all legal equivalents. Notwithstanding, none of the claims are intended to embrace subject matter that fails to satisfy the requirement of 35 U.S.C. § 101, 102, or 103, nor should they be interpreted in such a way.

## Claims

1. A fluid connector device (100), comprising:
a connector (102, 104) forming an internal chamber, an inlet, an outlet, and a post (105) that extends into the internal chamber, the post having a first post opening (142) and a second post opening (154); and
a slider assembly (110) comprising a first channel (132) and a first slider assembly opening (144), the slider assembly positioned in the internal chamber and movable, relative to the connector, between a first position in which the first slider assembly opening (144) is aligned with the first post opening (142) to permit a fluid flow between the first channel (132), the first slider assembly opening (144), the first post opening (142), and the outlet, and a second position in which the first slider assembly opening (144) is aligned with the second post opening (154) to permit the fluid flow between the first channel (132), the first slider assembly opening (144), the second post opening (154), and the outlet.

2. The fluid connector device of Claim 1, wherein the slider assembly (110) is movable along the post (105).

3. The fluid connector device of Claim 1, wherein the slider assembly (110) is rotatable, relative to the post (105).

4. The fluid connector device of Claim 1, wherein the post comprises a third post opening (156) and a fourth post opening (160), and wherein, in the second position, a second channel (148) of the slider assembly is fluidically coupled to the third post opening (156), and a third channel (150) of the slider assembly is fluidically coupled to the fourth post opening (160).

5. The fluid connector device of Claim 4, wherein the slider assembly (110) further comprises a first component (112) and a second component (114), and wherein the second channel (148) and the third channel (150) is formed between the first component and the second component.

6. The fluid connector device of Claim 1, wherein the slider assembly (110) further comprises a first component (112) and a second component (114), and wherein the first channel (132) is formed between the first component and the second component.

7. The fluid connector device of Claim 1, wherein any of the first post opening (142) and the second post opening (154) extend in a direction that is radially outward, relative to a longitudinal axis of the connector.

8. The fluid connector device of Claim 1, wherein the post (105) extends into a lumen of the slider assembly.

9. A blood collection system, comprising:
a blood collection device (40); and
the fluid connector device (100) of Claim 1 fluidically coupled to the blood collection device.

10. The blood collection system of Claim 9, wherein fluid flow through the second post opening (154) is resisted when the slider assembly is in the first position.

11. The blood collection system of Claim 9, wherein fluid flow through the first post opening (142) is resisted when the slider assembly is in the second position.

12. The blood collection system of Claim 9, wherein the slider assembly comprises an interface (118), and the slider assembly (110) is movable based on actuation of the interface.

13. A method for fluid connector device (100), the method comprising:
positioning a slider assembly (110) in an internal chamber of a connector (102, 104) having an inlet, an outlet, and a post, such that the slider assembly is movable between a first position and a second position, relative to the connector;
wherein, when the slider assembly is moved to the first position, a first slider assembly opening (144) aligns with a first post opening (142) to permit a fluid flow between a first channel (132) of the slider assembly, the first slider assembly opening (144), the first post opening (142), and the outlet, and when the slider assembly is moved to the second position, a first slider assembly opening (144) aligns with a second post opening (154) to permit the fluid flow between the first channel (132), the first slider assembly opening (144), the second post opening (154), and the outlet.

14. The method of Claim 13, wherein, when the slider assembly is moved to the second position, a second channel (148) of the slider assembly is fluidically coupled to a third post opening (156), and a third channel (150) of the slider assembly is fluidically coupled to a fourth post opening (160).

15. The method of Claim 14, further comprising forming the second channel (148) and the third channel (150) between a first component (112) of the slider assembly and a second component (114) of the slider assembly.
